# EUROPEAN PATENT SPECIFICATION

(11) **EP 3 053 559 B1**
(45) Date of publication and mention of the grant of the patent: **25.07.2018**
(21) Application number: 14847584.1
(22) Date of filing: 09.09.2014
(51) Int. Cl.: A61F 13/511, D04H 1/4374, A61F 13/47, A61F 13/15

(54) **ABSORBENT ARTICLE**
SAUGFÄHIGER ARTIKEL
ARTICLE ABSORBANT

(30) Priority: 30.09.2013 JP 2013204783
(43) Date of publication of application: 10.08.2016
(73) Proprietor: Daio Paper Corporation, Ehime 799-0492 (JP)
(72) Inventor: KURAMOCHI Mihoko, Sakura-shi Tochigi 329-1411 (JP)
(74) Representative: Neymeyr, Ulrich Theodor Paul
(86) International application number: PCT/JP2014/073732
(87) International publication number: WO 2015/045842

(56) References cited:
- EP-A1- 1 870 065
- JP-A- 2006 239 127
- JP-A- 2011 015 707
- JP-A- 2012 239 720

## Description

### Technical Field

The present invention relates to an absorbent article such as sanitary napkins, panty liners, incontinence pads, and disposable diapers for absorbing menstrual bloods, vaginal discharges, urine and the like, and particularly to an absorbent article provided with a concavo-convex pattern on a permeable front surface sheet.

### Background Art

Front surface materials for absorbent articles, in which an appropriate emboss pattern is imparted in accordance with various objects such as suppressing a wet feeling by reducing a contact area with a skin, or giving texture and improving a contact feeling have been commercialized. As materials of this kind, for example, the following Patent Literatures 1, 2 and the like can be cited.

In the following Patent Literature 1, an absorbent article that includes a permeable front surface sheet, an impermeable or water-repellent back surface sheet, and an absorber located between the both sheets, and is provided with an intermediate sheet between the front surface sheet and the absorber is disclosed, in the absorbent article, the permeable front surface sheet includes a first layer that forms a skin contact surface, and a second layer that is adjacent to the first layer and disposed on the absorber side, the first layer is made of a fiber that has a fineness of 2.2 dtex or less and hydrophilicity that is difficult to decrease due to liquid permeation, the second layer is constituted of a fiber having the fineness larger than the fineness of the constituent fiber of the first layer and contains a fiber of which hydrophilicity is difficult to decrease due to liquid permeation and a fiber of which hydrophilicity is likely to decrease due to the liquid permeation, the intermediate sheet includes a third layer located on the front surface sheet side and a fourth layer that is adjacent to the third layer and located on the absorber side, the fineness of the constituent fiber of the third layer is larger than the fineness of constituent fiber of the fourth layer, the fineness of the constituent fiber of the third layer is equal to or larger than the fineness of the constituent fiber of the second layer, the fineness of the constituent fiber of the fourth layer is smaller than the fineness of the constituent fiber of the second layer, the constituent fibers of the third layer and fourth layer are a fiber of which hydrophilicity is difficult to decrease due to liquid permeation, and an entirety or a part of the constituent fiber of the third layer is formed of a solid crimped fiber.

Further, in the following Patent Literature 2, a sheet for an absorbent article in which a first sheet having an outside layer and an inside layer is intermittently joined to a second sheet on the inside layer side is disclosed. In the sheet, a projected part protruding to the first sheet side formed between joining parts is connected to the sheet for an absorbent article, and a fiber that constitutes the outside layer of the first sheet is a fiber thinner than the fiber that constitutes the inside layer. Patent Literature 3 discloses an absorbent article comprising:an absorber interposed between a permeable front surface sheet and a back surface sheet; and a hydrophilic second sheet disposed between the permeable front surface sheet and the absorber; wherein the permeable front surface sheet is formed into a laminate structure that includes an hydrophobic non woven fiber layer that constitutes a skin contact surface layer, and a plastic film layer that is adjacently laminated on a non-skin surface side of the non woven layer, and is embossed into a concavo-convex shape formed of many raised parts protruding to a skin side.

### Citation List

### Patent Literature

Patent Literature 1: Japanese Patent No. 4566059
Patent Literature 2: JP 2009-50538 A
Patent Literature 3: EP 1 870 065 A1

### Summary of the Invention

### Technical Problem

In the absorbent article described in Patent Literature 1, by imparting a gradient of fineness and a gradient of strength of durability of hydrophilicity to the front surface sheet and the intermediate sheet each formed into a two-layered structure, an excellent liquid return prevention property is imparted and a touch feeling is improved. However, the absorbent article is not formed into a structure that can maintain a concavo-convex shape of an emboss pattern when the emboss pattern is imparted to the front surface sheet.

In the same manner also in the sheet for absorbent article described in Patent Literature 2, it is disclosed to impart a gradient of fineness to an inside layer and an outside layer to impart comfortable touch feeling or the like. However, while a projected part collapses or wilts due to body pressure or the like to deteriorate touch feeling, there is no disclosure of a technology for maintaining the shape retention property of projected part or for making it difficult to wilt.

Further, an absorbent article that, while maintaining the shape retention property of a concavo-convex shape on a surface, combines sensory performances such as skin touch feeling and uneven feeling, and absorption performance such as a return amount of body fluid in a balanced manner has not been developed.

There, a principal problem of the present invention is to provide an absorbent article in which, while maintaining excellent skin touch feeling, the concavo-convex shape due to a surface emboss processing is made easier to maintain, the skin touch feeling or the uneven feeling is made excellent and absorption performance is improved.

### Solution to Problem

As an invention according to claim 1 for solving the above-described problem, an absorbent article that includes an absorber interposed between a permeable front surface sheet and a back surface sheet and a hydrophilic second sheet disposed between the permeable front surface sheet and the absorber is provided, in which the permeable front surface sheet is formed into a laminate structure that includes a thin fiber layer made of a thin fiber that constitutes a skin contact surface and has a fineness of less than 2.0 dtex, and a crimped fiber layer made of a crimped fiber that is adjacently laminated on a non-skin surface side of the thin fiber layer and has the fineness of from 3.0 to 5.0 dtex, and is embossed into a concavo-convex shape formed of many raised parts protruding to a skin side.

According to the invention described in claim 1, the permeable front surface sheet is formed of a laminate structure that includes a thin fiber layer constituting a skin contact surface layer and a crimped fiber layer adjacently laminated on a non-skin surface side, and is embossed into a concavo-convex shape formed of many raised parts protruding to a skin side. Thus, since the permeable front surface sheet contains the crimped fiber layer, the raised parts are clearly formed on a skin side, and the permeable front surface sheet becomes rich in a cushioning property. Further, since due to the crimped fiber layer, an embossed concavo-convex shape can be firmly retained, the concavo-convex shape of the permeable front surface sheet becomes easy to retain, a structure that is difficult to wilt even when exposed to the body pressure is formed, an apparent uneven feeling of a front surface becomes clear and excellent skin touch feeling and cushioning property can also be apparently generated.

At this time, the crimped fiber layer does not come into direct contact with a skin and the touch feeling becomes smooth even when the front surface sheet comes into contact with and rub the skin because a thin fiber layer that constitutes a skin contact surface layer is formed on a skin surface side of the crimped fiber layer and the thin fiber layer is constituted of a thin fiber having the fineness of less than 2.0 dtex.

Further, by constituting the crimped fiber layer with the crimped fiber having the fineness of from 3.0 to 5.0 dtex, as obvious from a test result that will be described below, by providing, on a non-skin surface side of the thin fiber layer, a crimped fiber layer that uses the fiber having the fineness larger than that of the thin fiber layer and uses a fiber that is provided with a crimp property by heating, the sensory performance such as the skin touch feeling and the uneven feeling and an absorption performance such as the return amount of the body fluid can be provided in a balanced manner.

As an invention according to claim 2, the absorbent article according to claim 1 in which the second sheet made of a fiber having the fineness of from 2.0 to 6.0 dtex is provided.

In the invention described in claim 2, by using a little thicker fiber of the fineness of from 2.0 to 6.0 dtex as the second sheet, voids are easily formed between the fibers, and the body fluid is easily transferred to the absorber side. Thus, it is difficult to generate a liquid residue on the front surface side and a dry touch feeling can be maintained.

As an invention according to claim 3, the absorbent article according to any one of claims 1 and 2 in which a hydrophilizing agent is coated on each of the fibers that constitute the permeable front surface sheet and the second sheet, and strength of durability of the hydrophilizing agent has the relationship of thin fiber layer ≤ crimped fiber layer < second sheet is provided.

In the invention according to claim 3, in the case where each of the permeable front surface sheet (thin fiber layer and crimped fiber layer) and the second sheet is imparted with the hydrophilicity by coating a hydrophilizing agent on the fiber, by constituting such that the strength of the durability during body fluid passage of the hydrophilizing agent satisfies the predetermined relationship, the body fluid is easily moved to the absorber, and by preventing the body fluid absorbed by the absorber from rebounding, the dry touch feeling of the front surface side is improved.

### Advantageous Effects of Invention

As was described above, according to the present invention, while maintaining excellent touch feeling, the concavo-convex shape due to the surface embossing process can be maintained, the skin touch feeling and the uneven feeling become excellent, and the absorption performance becomes excellent.

### Brief Description of Drawings

[FIG.1] FIG. 1 is a partially broken perspective view of a sanitary napkin 1 according to the present invention.
[FIG.2] FIG. 2 is a cross-sectional view of a permeable front surface sheet 3 and a second sheet 6.
[FIG.3] FIG. 3 is a side surface view of a manufacturing apparatus 20 of the permeable front surface sheet 3 and second sheet 6.
[FIG. 4] FIG. 4 is an engagement view of a first embossing roll 21 and a second embossing roll 22.
[FIG. 5] FIG. 5 is a test result of a return amount.

### Description of Embodiments

Hereinafter, an embodiment of the present invention will be described in more detail with reference to the drawings.

### (Structure of Sanitary Napkin 1)

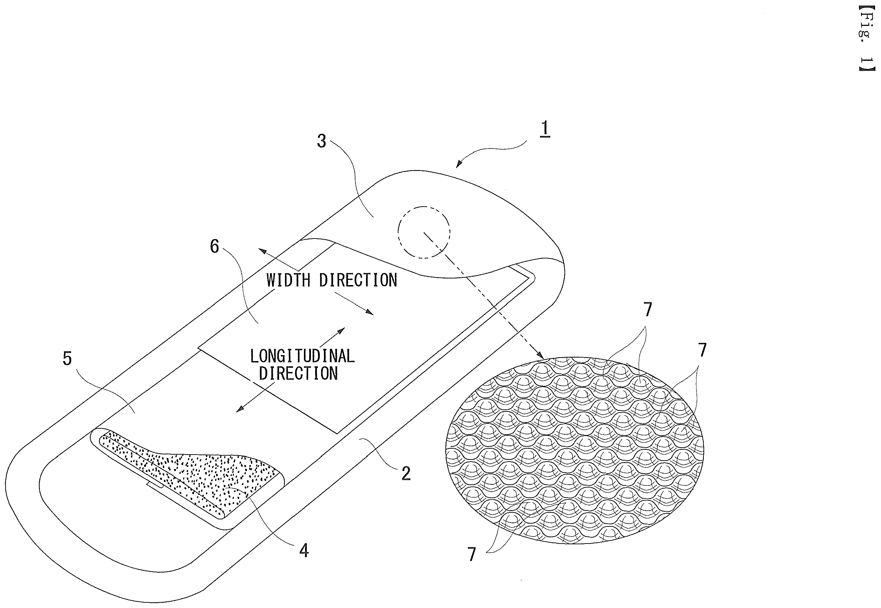

A sanitary napkin 1 is supplied to use mainly in panty liners, sanitary napkins, vaginal discharge sheets, incontinence pads and the like and has a structure in which, as shown, for instance, in FIG. 1, an absorber 4 is disposed between an impermeable back surface sheet 2 and a permeable front surface sheet 3 (hereinafter, simply referred to also as a surface sheet) or, as shown in the same drawing, a hydrophilic second sheet 6 is disposed between the impermeable back surface sheet 2 and the absorber 4 surrounded by a crepe paper 5. In the surroundings of the absorber 4, the impermeable back surface sheet 2 and the front surface sheet 3 are joined by adhering means such as a hot-melt adhesive or the like. It should be noted that as long as the sanitary napkin 1 according to the present invention includes at least the permeable front surface sheet 3 and the second sheet 6, sanitary napkins of well-known forms such as one provided with a side nonwoven fabric along a longitudinal direction of each of both side parts on a skin surface side or one provided with a wing-like flap fixed so as to engulf a crotch part of an underwear during wearing on each of both side parts can be widely used.

For the impermeable back surface sheet 2, a sheet material having at least water shieldability such as polyethylene, polypropylene or the like can be used. However, other than these, a nonwoven fabric in which impermeability is substantially secured by interposing a water-proof film (in this case, the impermeable back surface sheet is formed with the water-proof film and the nonwoven fabric) and the like can be used. In recent years, there is a tendency to preferably use a sheet material having moisture permeability from the viewpoint of stuffiness prevention. As the water-shielding and permeable sheet material, a microporous sheet obtained by stretching a sheet in a uniaxial or biaxial direction after the sheet is molded by melt-kneading an inorganic filler in an olefinic resin such as polyethylene or polypropylene can be suitably used.

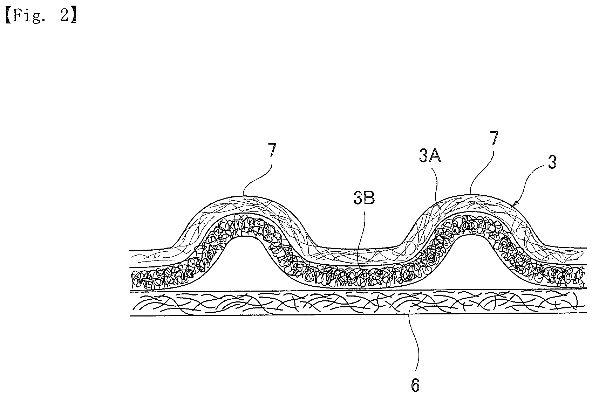

The front surface sheet 3, as shown in FIG. 2, uses a multilayered sheet that has a thin fiber layer 3A constituting at least a skin contact surface layer and a crimped fiber layer 3B laminated more adjacently to a non-skin contact surface side than to the thin fiber layer 3A and that is embossed into a concavo-convex shape formed of many raised parts 7, 7 ··· protruding to a skin side. It should be noted that the front surface sheet 3 will be more specifically described below.

The absorber 4 that is interposed between the impermeable back surface sheet 2 and the permeable front surface sheet 3 is made of, for example, a fluff-like pulp and a superabsorbent polymer. As the superabsorbent polymer, a superabsorbent polymer granular powder (SAP) or a superabsorbent polymer fiber (SAF) may be used. As the pulp, a chemical pulp obtained from wood, cellulose fibers such as dissolving pulp and synthetic cellulose fibers such as rayon and acetate can be used. A softwood pulp having a fiber length longer than that of a hardwood pulp is suitably used from the viewpoint of function and price. According to a manufacturing method of the absorber 4, the absorber 4 may be desirably formed into a fiber stacking pulp so as to be rich in flexibility but may be formed into an air-laid absorber of which volume can be reduced.

The hydrophilic second sheet 6 disposed between the permeable front surface sheet 3 and the absorber 4 may only have a hydrophilic property to the body fluid. Specifically, by using recycled fiber such as rayon, cupra, or the like, or natural fiber such as cotton or the like, fibers having a hydrophilic property in the raw material itself can be used, or fibers to which a hydrophilic property is imparted by surface treating synthetic fibers such as olefin-based fibers such as polyethylene or polypropylene, polyester-based fibers, or polyamide-based fibers with a hydrophilizing agent can be used. The latter is preferably used.

### (Structure of Front Surface Sheet 3)

The front surface sheet 3 includes, as shown in FIG. 2, a two-layered structure that is made of a thin fiber layer 3A constituting a skin contact surface layer and a crimped fiber layer 3B laminated more adjacently to a non-skin surface side than to the thin fiber layer 3A and that is embossed into a concavo-convex shape formed of many raised parts 7, 7 ··· protruding to a skin side.

As a raw material fiber that constitutes the thin fiber layer 3A, a raw material fiber made of a thin fiber having the fineness of less than 2.0 dtex, preferably of about exceeding 1.1 dtex and less than 2.0 dtex is used. When the thin fiber having the fineness in this range is used, the touch feeling when the fiber comes into contact with a skin becomes soft, the rubbing feeling is reduced and the skin touch feeling is improved, and the absorption performance is made excellent.

As a raw material fiber that constitutes the thin fiber layer 3A, for example, other than synthetic fibers such as olefin based fibers such as polyethylene or polypropylene, polyester based fibers, and polyamide based fibers, recycled fibers such as rayon and cupra, and natural fibers such as cotton can be used, and a nonwoven fabric obtained according to an appropriate processing method such as an air-through method, a spun lace method, a spun bond method, a thermal bond method, a melt blown method, a needle punch method or the like can be used. Among these processing methods, the spun lace method is excellent in a point of being rich in flexibility, the spun bond method is excellent in a point of being rich in a drape property, and the air-through method and the thermal bond method are excellent in a point of being bulky and soft. A fiber of the nonwoven fabric may be either one of a long fiber and a short fiber. However, the short fiber is preferably used to show texture of toweling. Further, an olefin-base fiber such as polyethylene or polypropylene having a relatively low melting point is preferably used to make it easy to apply an embossing process. Still further, a composite fiber such as a core/sheath type fiber in which a fiber having a high melting point is used as a core and a fiber having a low melting point is used as a sheath, a side-by-side type fiber, a divided type fiber or the like can be preferably used.

As a raw material fiber that constitutes the crimped fiber layer 3B, a raw material fiber made of a crimped fiber is used. In order to impart crimpability, it is preferable that a core-sheath structure having synthetic fibers having different heat shrinkage temperatures as a core and a sheath be formed, a core part be made eccentric with respect to a sheath part, and the fiber be three-dimensionally crimped by heat treatment. In particular, it is preferable to use polyethylene terephthalate (PET) having a melting point of about 255°C in the core part, and polyethylene (PE) having a melting point of from about 95 to 140°C is used in the sheath part. When the PET having higher strength of stiffness than that of other fibers is used in the core part, the stiffness of the crimped fiber layer 3B can be increased, by compensating small stiffness of the thin fiber layer 3A, a cushion property and shape retention of a concavo-convex shape of the front surface sheet 3 can be made more excellent. Further, the side-by-side composite fiber obtained by adhering synthetic resins having different heat shrinkage temperatures can also be used by heat treating, or by imparting a zigzag crimp or a spiral crimp by stretching.

When the crimped fiber layer 3B is constituted of a crimping raw material fiber, the crimped fiber layer becomes bulky and shows stiffness. Therefore, even when a superficial thin fiber layer 3A is a soft fiber layer constituted of a thin fiber, the front surface sheet 3 retains firmly a concavo-convex shape by embossing, the cushion property due to the concavo-convex shape is difficult to degrade and becomes difficult to wilt even when exposed to the body pressure. Therefore, a dry touch feeling can be maintained because a smooth touch feeling due to the thin fiber layer 3A is maintained, and a contact area with a skin due to the concavo-convex shape can be reduced.

As the fineness of the raw material fiber that constitutes the crimped fiber layer 3B, the fineness larger than the fineness of the thin fiber layer 3A is used. Specifically, the fineness of from 3.0 to 5.0 dtex, preferably of from about 3.0 to 4.5 dtex is used. When the fiber having the fineness larger than the fineness of the thin fiber layer 3A is used, the shape retention of the concavo-convex shape of the permeable front surface sheet 3 becomes excellent, the cushion property can be more surely retained, and absorption performance of the body fluid also becomes excellent. The detail will be described in the later examples.

As the fineness of the raw material fiber that constitutes the second sheet 6, the fineness of from 2.0 to 6.0 dtex, preferably of about from 3.0 to 4.0 dtex is used. When a little thicker fiber, preferably a fiber of the fineness larger than the fineness of permeable front surface sheet 3 is used, a gap tends to be generated between fibers, and the body fluid that passed through the permeable front surface sheet 3 moves easily to the absorber 4 side. Therefore, the liquid is difficult to remain on a front surface side and a dry touch feeling can be maintained.

It should be noted that the relationship of the finenesses of the raw material fibers that constitute the front surface sheet 3 (thin fiber layer 3A and crimped fiber layer 3B) and the second sheet 6 preferably satisfies thin fiber layer 3A < crimped fiber layer 3B ≤ second sheet 6. When a fineness gradient in which the fineness becomes higher toward the absorber 4 side is disposed and the gaps between the fibers are made gradually larger, the body fluid can be rapidly transferred to the absorber 4 side.

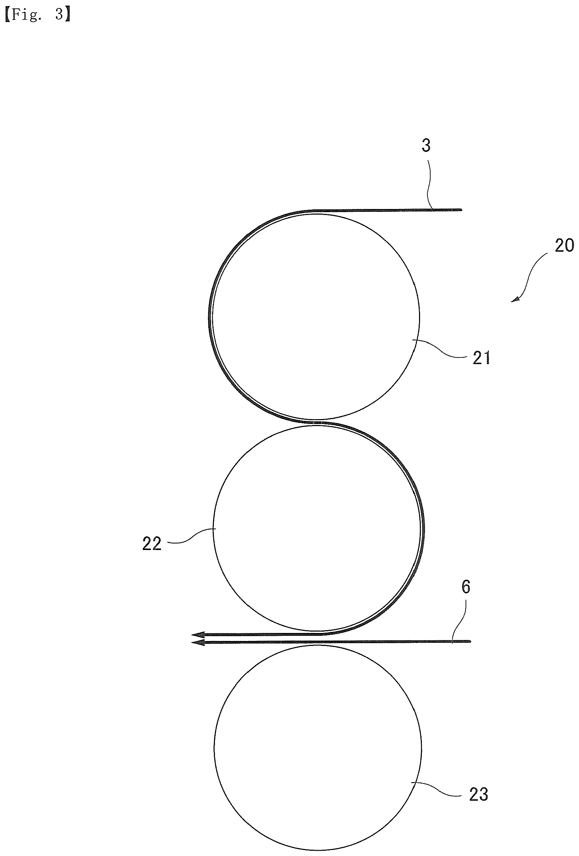

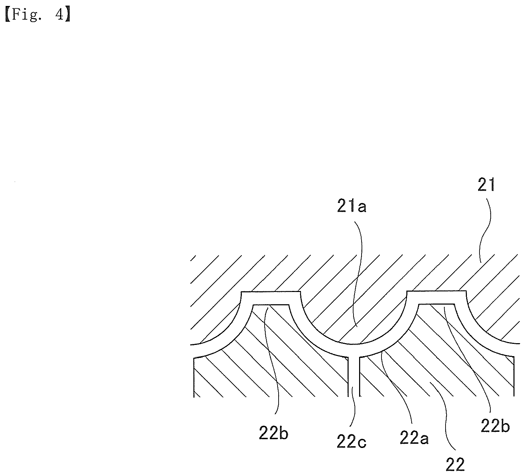

When an embossing process is applied to the permeable front surface sheet 3, as shown in FIG. 3 and FIG. 4, a manufacturing apparatus 20 is preferably used, in which by using a first embossing roll 21 on which many projected parts 21a, 21a ··· are arranged and a second embossing roll 22 on which many recessed parts 22a, 22a ··· are formed corresponding to the projected parts 21a, when the front surface sheet 3 in which the thin fiber layer 3A and the crimped fiber layer 3B are laminated is passed between these first embossing roll 21 and the second embossing roll 22, by engaging the projected part 21a and the recessed part 22a, an emboss pattern is imparted.

After that, by passing the second sheet 6 transferred from a separate path in a laminated state between the second embossing roll 22 and a flat roll 23, the front surface sheet 3 and the second sheet 6 are joined and integrated. When joining these, a hot-melt adhesive or the like is coated on an external surface (external surface of the crimped fiber layer 3B) of the front surface sheet 3 corresponding to the projected part 22b of the second embossing roll 22 and the front surface sheet 3 and the second sheet 6 are adhered, or when engaging with the flat roller 23, by heating the projected part 22b of the second embossing roll 22, the front surface sheet 3 and the second sheet 6 may be heat-sealed.

In order to more clearly form the concavo-convex shape by embossing, an aperture 22c is formed at an apex of the recessed part 22a of the embossing roll 22 on a recessed side, and by suctioning by a vacuum from the aperture 22c during engaging with the projected part 21a to generate a negative pressure, the front surface sheet 3 can be made easy to deform along the recessed part 22a.

When the concavo-convex shape is imparted to the front surface sheet 3 by using the manufacturing apparatus 20, as shown in FIG. 2, the front surface sheet 3 is formed into many raised parts 3a, 3a ... bulging on a skin side, and a space is formed between the front surface sheet 3 and the second sheet 6 in the raised part 3. By joining the front surface sheet 3 and the second sheet 6 while having the space, the cushioning property of the front surface sheet 3 is increased and the shape of the raised part 3a becomes easy to maintain. At this time, the crimped fiber layer 3B is interposed on the front surface sheet 3. Therefore, even when the space is exposed to the body pressure that may collapse the space, due to the strength of the stiffness of the crimped fiber, resiliency for returning to an initial form is high, and the cushioning property becomes rich.

Each of the fibers that constitute the front surface sheet 3 (thin fiber layer 3A and crimped fiber layer 3B) and the second sheet 6 is preferably a hydrophobic fiber coated with a hydrophilizing agent, and the strength of the durability of the hydrophilizing agent at this time is preferably constituted so as to satisfy the relationship of thin fiber layer 3A ≤ crimped fiber layer 3B < second sheet 6. The strength of the durability of the hydrophilizing agent means a degree that maintains a state in which the hydrophilizing agent is fixed to the fiber without falling off the fiber surface and flowing off together with the liquid during liquid passage, and the small strength means that the hydrophilizing agent is likely to flow off together with the liquid during liquid passage.

As the hydrophilizing agent, for example, an anionic surfactant, a carboxylate, an acylated hydrolyzed protein, a sulfonate, a sulfuric acid ester salt, a phosphoric acid ester salt, a nonionic surfactant, a polyoxyethylene base surfactant, a carboxylic acid ester, carboxylic acid amide, a polyalkylene oxide block copolymer, a cationic surfactant, a quaternary ammonium salt, an amphoteric surfactant, an imidazolium derivative or the like can be used. Other than these, any hydrophilizing agent that is known as the hydrophilizing agent that is coated on the fiber may be applied.

As a coating method of the hydrophilizing agent, for example, coating by spraying, coating by gravure printing or flexo printing, curtain coating by various coaters can be used. Further, the hydrophilizing agent may be kneaded in the stage of the fiber. The hydrophilicity can be controlled by controlling a coating amount of the hydrophilizing agent. In order to make the body fluid readily infiltrate to the absorber 4 side, the hydrophilicity is preferably controlled such that the relationship of thin fiber layer 3A ≤ crimped fiber layer 3B < second sheet 6 may be satisfied.

Further, when the durability of the hydrophilizing agent can be controlled, as an adhesive resin or a catalyst, an acrylic aqueous resin, a gum-base latex, a urethane resin, a polyester-base resin, a polyvinyl resin and the like are used together, and addition amounts of these are controlled.

When the strength of the durability of the hydrophilizing agent on a front surface side is attenuated, the affinity with the fiber of the hydrophilizing agent on the front surface side is decreased, the hydrophilizing agent attached to the fiber during one time liquid passage is flowed out together with the body fluid, and this part tends to be a hydrophobic fiber. At this time, when the strength of the durability of the hydrophilizing agent of the thin fiber layer 3A disposed on a skin contact surface layer is made smallest, the body fluid is made easy to move to the absorber 4 side, the body fluid absorbed by the absorber 4 becomes difficult to return due to a water-repellent action of the hydrophobic fiber, and the dry touch feeling on the front surface side is improved.

### EXAMPLES

In order to perform a sensory evaluation of the skin touch feeling and the uneven feeling and an absorption performance evaluation of the return amount of the body fluid of the present sanitary napkin, the following tests were carried out.

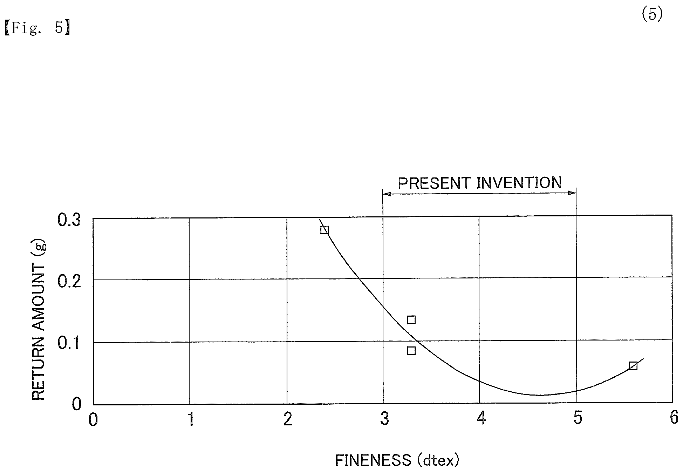

Specifications of the sanitary napkins used in the test and the results are as shown in Table 1 and FIG. 5.

**[Table 1]**

| | | Example 1 | Example 2 | Comparative Example 1 | Comparative Example 2 |
|---|---|---|---|---|---|
| Specification of permeable top sheet 3 | | Air-through 25 gsm | | | |
| Specification of thin fiber layer 3A | Basis weight (gsm) | 10 | | | |
| | Kind | PP/PE (concentric core type) | | | |
| | Fineness (dtex) | 1.4 | 1.1 | 1.1 | 1.1 |
| Specification of crimped fiber layer 3B | Basis weight (gsm) | 15 | | | |
| | Kind | PP/PE (eccentric core type) | | | |
| | Fineness (dtex) | 3.3 | 3.3 | 2.4 | 5.6 |
| Specification of second sheet 6 | | Air-through 18 gsm, PP/PE, 5.6 dtex | | | |
| Specification of absorber 4 | | Air-laid | | | |
| Sensory performance | Touch feeling | Excellent | Good | Good | Poor |
| | Uneven feeling | Good | Good | Poor | Good |
| Absorption performance | Result of absorption performance evaluation | Good | Poor | Bad | Poor |
| | Return amount (g) | 0.083 | 0.133 | 0.278 | 0.111 |

As an evaluation method of the skin touch feeling, subjects relatively evaluated the touch feeling when a surface of each of the above-described 4 samples was touched with a hand. As the uneven feeling, an appearance of an apparent concavo-convex state of a front surface was relatively evaluated for each of the above-described 4 samples. Regarding evaluation criteria of the skin touch feeling and touch feeling, among the 4 samples, a sample that was determined to be most excellent by 4 or more subjects among 10 subjects was denoted as excellent, a sample that was determined to be most excellent by 2 or more and 3 or less subjects was denoted as good, a sample that was determined to be most excellent by one subject was denoted as poor, and a sample that was determined to be most excellent by 0 subject was denoted as bad. Regarding the return amount, 1 cc of artificial menstrual blood of 37°C was injected, after an interval of 3 minutes, 1 cc of the artificial menstrual blood of 37°C was injected at the same place once again, after an interval of 1 minute, a top surface thereof was covered with a filter paper, from the above thereof, a weight of 5 g/cm² was disposed for 5 minutes, and a change of weight of the filter paper was measured as the return amount. The artificial menstrual blood that contains, in 1 L, 100 ml of glycerin, 4.6 g of CMC (sodium carboxymethylcellulose), 875 ml of purified water, 10 g of sodium chloride, and 10.7 g of sodium carbonate was used.

As the result, according to the sensory evaluation such as the skin touch feeling and uneven feeling, it was shown, as shown in Table 1, that the sample having the fineness of the crimped fiber layer 3B of 3.3 dtex was most excellent. Further, according to the absorption performance evaluation regarding the return amount of the body fluid, it was shown, as shown in FIG. 5, that the return amount had a minimum value in the neighborhood of 4.5 dtex of the fineness of the crimped fiber layer 3B. From these results, the crimped fiber layer 3B having the fineness of from 3.0 to 5.0 dtex has excellent sensory performance such as the skin touch feeling and the uneven feeling and has excellent absorption performance that can suppress the return amount of the body fluid low, that is, combines the sensory performance and the absorption performance in a good balance. Further, preferably, the crimped fiber layer 3B having the fineness of from 3.0 to 4.5 dtex where the return amount is in the neighborhood of the minimum value and the skin touch feeling and the uneven feeling are excellent is better.

Further, in the comparison of Example 1 and Example 2, when the fineness of the thin fiber layer 3A was changed from 1.4 dtex to 1.1 dtex, while the sensory performance maintained substantially excellent state, the absorption performance was slightly deteriorated because the return amount increased from 0.083 g to 0.133 g. Therefore, the fineness of the thin fiber layer 3A is preferably less than 2.0 dtex and larger than 1.1 dtex.

### [Example of Other Forms]

(1) In the example of a form, the permeable front surface sheet 3 was formed into the two-layered structure that was made of the thin fiber layer 3A constituting the skin contact surface layer and the crimped fiber layer 3B that was adjacently laminated on the non-skin surface side of the thin fiber layer 3A. However, a composite layer sheet of a three-layered structure or more obtained by laminating other sheet material on the non-skin surface side of the crimped fiber layer may be formed.
(2) In the example of a form, while the permeable front surface layer 3 was formed into the composite layer structure made of the thin fiber layer 3A and the crimped fiber layer 3B, by mixing the thin fiber of the fineness of less than 2.0 dtex of the thin fiber 3A and the crimped fiber of the crimped fiber layer 3B, a single-layered structure of one layer may be formed. Thus, while the shape retention of the concavo-convex shape due to the embossing process is deteriorated compared with the composite layer structure, since a fiber density of a front surface does not become denser, the absorptivity of the body fluid becomes higher and the liquid can be prevented from remaining on the front surface. A mixing ratio is preferably set to fiber of the thin fiber layer 3A: fiber of the crimped fiber layer 3B = 3:7 to 7:3.

### Reference Signs List

- 1: sanitary napkin
- 2: impermeable back surface sheet
- 3: permeable front surface sheet
- 3A: thin fiber layer
- 3B: crimped fiber layer
- 4: absorber
- 5: crepe paper
- 6: second sheet
- 7: raised part
- 20: manufacturing apparatus
- 21: first embossing roll
- 22: second embossing roll
- 23: flat roll

## Claims

1. An absorbent article comprising:
an absorber interposed between a permeable front surface sheet and a back surface sheet; and
a hydrophilic second sheet disposed between the permeable front surface sheet and the absorber,
wherein the permeable front surface sheet is formed into a laminate structure that includes a thin fiber layer made of a thin fiber that constitutes a skin contact surface layer and has a fineness of less than 2.0 dtex, and a crimped fiber layer made of a crimped fiber that is adjacently laminated on a non-skin surface side of the thin fiber layer and has the fineness of from 3.0 to 5.0 dtex, and is embossed into a concavo-convex shape formed of many raised parts protruding to a skin side.

2. The absorbent article according to claim 1, wherein the second sheet is made of a fiber having the fineness of from 2.0 to 6.0 dtex.

3. The absorbent article according to any one of claims 1 and 2, wherein a hydrophilizing agent is coated on each of the fibers that constitute the permeable front surface sheet and the second sheet, and strength of durability of the hydrophilizing agent have the relationship of thin fiber layer ≤ crimped fiber layer < second sheet.

## Patentansprüche

1. Absorbierender Artikel, umfassend:
einen zwischen eine permeable Vorderseitenoberflächenlage und eine Rückseitenoberflächenlage eingefügten Absorber; und
eine hydrophile zweite Lage, die zwischen der permeablen Vorderseitenoberflächenlage und dem Absorber angeordnet ist,
wobei die permeable Vorderseitenoberflächenlage zu einer Laminatstruktur ausgebildet ist, die eine aus einer dünnen Faser hergestellte Dünnfaserschicht, die eine mit der Haut in Kontakt gelangende Oberflächenschicht bildet und eine Faserfeinheit von weniger als 2,0 dtex aufweist, und eine aus einer gekräuselten Faser hergestellte Kräuselfaserschicht, die benachbart auf eine nicht mit der Haut in Kontakt gelangende Oberflächenseite der Dünnfaserschicht laminiert ist und die Faserfeinheit von 3,0 bis 5,0 dtex aufweist und in eine konkav-konvexe Form geprägt ist, die aus zahlreichen erhabenen, zu einer Hautseite hervortretenden Teilen gebildet ist, umfasst.

2. Absorbierender Artikel nach Anspruch 1, wobei die zweite Lage aus einer Faser, die eine Faserfeinheit von 2,0 bis 6,0 dtex aufweist, hergestellt ist.

3. Absorbierender Artikel nach einem der Ansprüche 1 und 2, wobei ein hydrophilisierendes Mittel auf jede der Fasern, die die permeable Vorderseitenoberflächenlage und die zweite Lage bilden, aufgetragen ist und die Widerstandskraft des hydrophilisierenden Mittels das Verhältnis Dünnfaserschicht ≤ Kräuselfaserschicht < zweite Lage aufweist.

## Revendications

1. Article absorbant comprenant :
un agent absorbeur interposé entre une feuille de surface avant perméable et une feuille de surface arrière ; et
une seconde feuille hydrophile disposée entre la feuille de surface avant perméable et l'agent absorbeur,
dans lequel la feuille de surface avant perméable est formée au sein d'une structure stratifiée qui inclut une couche de fibre fine constituée d'une fibre fine qui constitue un couche de surface de contact avec la peau et possède une fibre plissée qui est stratifiée de manière adjacente sur un coté de surface de non peau de la couche de fibre fine et présente une finesse inférieure à 2,0 dtex, et une couche de fibre plissée constituée d'une fibre plissée qui est stratifiée de manière adjacente sur un coté de surface de non peau de la couche de fibre fine et présente une finesse comprise entre 3,0 et 5,0 dtex, et est en relief au sein d'une forme concavo-convexe formée de nombreuses parties surélevées faisant saillie sur un coté de peau.

2. Article absorbant selon la revendication 1, dans lequel la seconde feuille est constituée d'une fibre présentant une finesse comprise entre 2,0 et 6,0 dtex.

3. Article absorbant selon l'une quelconque des revendications 1 et 2, dans lequel un agent conférant un caractère hydrophile est revêtu sur chacune des fibres qui constitue la feuille de surface avant perméable et la seconde feuille, et une force de durabilité de l'agent conférant un caractère hydrophile possèdent la relation suivante : couche de fibre mince ≤ couche de fibre plissée < seconde feuille.
